(19) Europäisches Patentamt
European Patent Office
Office européen des brevets

(11) Numéro de publication : **0 607 076 A1**

# (12) DEMANDE DE BREVET EUROPEEN

(21) Numéro de dépôt : **94400057.9**

(22) Date de dépôt : **11.01.94**

(51) Int. Cl.[5] : **C07D 487/04,** A61K 31/415, A61K 31/445, A61K 31/495, A61K 31/535, A61K 31/54, A61K 31/55, C07D 519/00, // (C07D487/04, 235:00, 235:00), (C07D519/00, 498:00, 487:00)

(30) Priorité : **15.01.93 FR 9300337**
**22.07.93 FR 9309013**

(43) Date de publication de la demande :
**20.07.94 Bulletin 94/29**

(84) Etats contractants désignés :
**AT BE CH DE DK ES FR GB GR IE IT LI LU MC NL PT SE**

(71) Demandeur : **SYNTHELABO**
**22, Avenue Galilée**
**F-92350 Le Plessis Robinson (FR)**

(72) Inventeur : **George, Pascal**
**19 rue des Quatre Vents**
**F-78730 St Arnoult en Yvelines (FR)**

Inventeur : **De Peretti, Danielle**
**14 rue Ampere**
**F-92160 Antony (FR)**
Inventeur : **Roy, Jocelyne**
**6 rue de l'Hôtel de Ville**
**F-91130 Ris-Orangis (FR)**
Inventeur : **Schmitt, Jean-Paul**
**13 rue Victor Hugo**
**F-91290 Arpajon (FR)**
Inventeur : **Sevrin, Mireille**
**73 rue Raymond Losserand**
**F-75014 Paris (FR)**

(74) Mandataire : **Ludwig, Jacques et al**
**SYNTHELABO,**
**Service Brevets,**
**B.P. 72**
**F-92352 Le Plessis Robinson Cédex (FR)**

(54) **Dérivés de 9h-imidazo 1,2-a benzimidazole-3-acétamide à activité GABA.**

(57) Composé répondant à la formule générale (I)

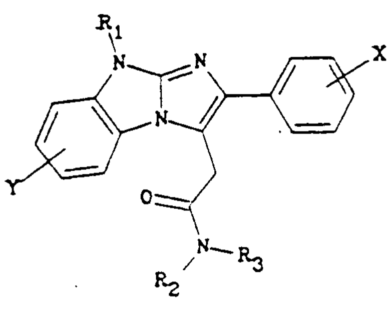

(I)

dans laquelle X représente un ou plusieurs atomes ou groupes choisis parmi l'hydrogène, les halogènes, les groupes alkyle, trifluorométhyle, alcoxy, alkylthio, méthylsulfonyle, cyano, aminocarbonyle et carboxy, Y représente un ou plusieurs atomes ou groupes choisis parmi l'hydrogène, les halogènes, les groupes alkyle, trifluorométhyle, méthoxy et trifluorométhoxy, $R_1$ représente l'hydrogène ou un groupe alkyle, phénylméthyle, 2-phényléthyle, acétyle ou alcoxycarbonyle, $R_2$ et $R_3$ représentent chacun l'hydrogène ou un groupe alkyle éventuellement substitué, prop-2-ényle, prop-2-ynyle, phényle, 1-(phénylméthyl)pipéridin-4-yle, 1-[(cyclohexen-1-yl)méthyl]pipéridin-4-yle, ou bien $R_2$ et $R_3$ forment avec l'atome d'azote qui les porte un hétérocycle éventuellement substitué.
Ils possèdent des propriétés hypnotiques, anxiolytiques et anticonvulsivantes et, par conséquent, peuvent être utilisés pour le traitement d'affections liées aux désordres de la transmission GABAergique, tels que l'anxiété, les troubles du sommeil, l'épilepsie, la spasticité, les contractures musculaires, les troubles cognitifs, les troubles du sevrage vis-à-vis de l'alcoolisme, etc. Ils peuvent être utilisés dans

EP 0 607 076 A1

la prémédication et comme anesthésiques généraux pour l'induction et/ou le maintien de l'anesthésie, ou comme anesthésiques locaux, éventuellement associés à d'autres anesthésiques et/ou des myorelaxants et/ou des analgésiques.

La présente invention a pour objet des dérivés de 9*H*-imidazo[1,2-*a*]benzimidazole-3-acétamide, leur préparation et leur application en thérapeutique.

Les composés de l'invention répondent à la formule générale (I)

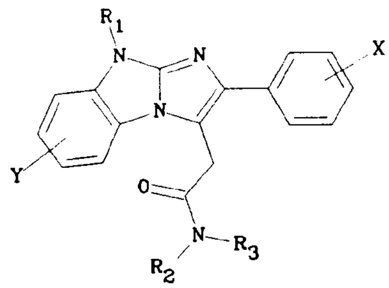

(I)

dans laquelle

X représente un ou plusieurs atomes ou groupes choisis parmi l'hydrogène, le fluor, le chlore, le brome et les groupes ($C_1$-$C_3$)alkyle, trifluorométhyle, ($C_1$-$C_3$)alcoxy, ($C_1$-$C_3$)alkylthio, méthylsulfonyle, cyano, éthoxycarbonyle, aminocarbonyle et carboxy,

Y représente un ou plusieurs atomes ou groupes choisis parmi l'hydrogène, le fluor, le chlore, le brome et les groupes ($C_1$-$C_4$) alkyle, trifluorométhyle, méthoxy et trifluorométhoxy, $R_1$ représente un atome d'hydrogène, un groupe ($C_1$-$C_3$)alkyle, un groupe phénylméthyle, un groupe 2-phényléthyle, un groupe acétyle ou un groupe ($C_1$-$C_3$) alcoxycarbonyle,

$R_2$ et $R_3$ représentent chacun, indépendamment l'un de l'autre, soit un atome d'hydrogène, soit un groupe ($C_1$-$C_5$)alkyle linéaire ou éventuellement ramifié ou cyclique, et éventuellement substitué par un ou plusieurs atomes de fluor, par un groupe méthoxy, par un groupe phénoxy, par un groupe diméthylamino, par un groupe phényle ou par un groupe imidazol-4-yle, soit un groupe prop-2-ényle, soit un groupe prop-2-ynyle, soit un groupe phényle, soit un groupe 1-(phénylméthyl)pipéridin-4-yle, soit un groupe 1-[(cyclohexen-1-yl)méthyl]pipéridin-4-yle,

ou bien

$R_2$ et $R_3$ forment ensemble, et avec l'atome d'azote qui les porte, un groupe pyrrolidin-1-yle, 3-éthoxypyrrolidin-1-yle, pipéridin-1-yle, 4-(phénylméthyl) pipéridin-1-yle, spiro(dioxolane-2,4'-pipéridin)-1'-yle, 3-(phénoxyméthyl)pipéridin-1-yle, 4-(phénoxyméthyl)pipéridin-1-yle, hexahydroazépin-1-yle, 4-méthylpipérazin-1-yle, 4-(phénylméthyl)pipérazin-1-yle, morpholin-4-yle ou thiomorpholin-4-yle.

Les composés de l'invention peuvent se présenter à l'état de bases libres ou de sels d'addition à des acides.

Parmi les composés de l'invention on préfère ceux dans la formule desquels X est en position 4 et représente un atome d'hydrogène ou de fluor ou un groupe méthyle, ceux dans la formule desquels Y est en position 6 et représente un atome d'hydrogène ou de fluor ou un groupe méthyle, ceux dans la formule desquels $R_1$ représente un atome d'hydrogène ou un groupe méthyle, et ceux dans la formule desquels $R_2$ et $R_3$ représentent chacun, indépendamment l'un de l'autre, un atome d'hydrogène ou un groupe méthyle.

Conformément à l'invention, on peut préparer ces composés selon un procédé illustré par le schéma 1 qui suit.

On fait réagir un dérivé de 9*H*-imidazo[1,2-*a*]benzimidazole de formule générale (II) (dans laquelle X, Y et $R_1$ sont tels que définis ci-dessus) avec le *N,N*-diméthylglyoxamide (que l'on prépare *in situ* au moyen de 2,2-diéthoxy-*N,N*-diméthylacétamide, comme décrit dans la demande de brevet EP-251859) dans un solvant protique tel que l'acide acétique à une température de 20 à 80°C.

On traite ensuite le dérivé d'α-hydroxyacétamide de formule générale (III) avec un polyhalogénure d'acide sulfurique ou phosphorique, par exemple le chlorure de thionyle ou l'oxychlorure de phosphore, ou tout autre agent équivalent, dans un solvant inerte, par exemple un solvant chloré ou éthéré tel que le dichlorométhane ou le tétrahydrofurane, à une température de 20 à 80°C, pour former le dérivé de α-halogénoacétamide correspondant, puis on fait réagir ce dernier soit avec un agent réducteur tel qu'un hydrure alcalin simple ou complexe, par exemple le borohydrure de sodium ou de potassium, dans un solvant protique, par exemple un alcool aliphatique tel que le méthanol ou l'éthanol, ou dans un

Schéma 1

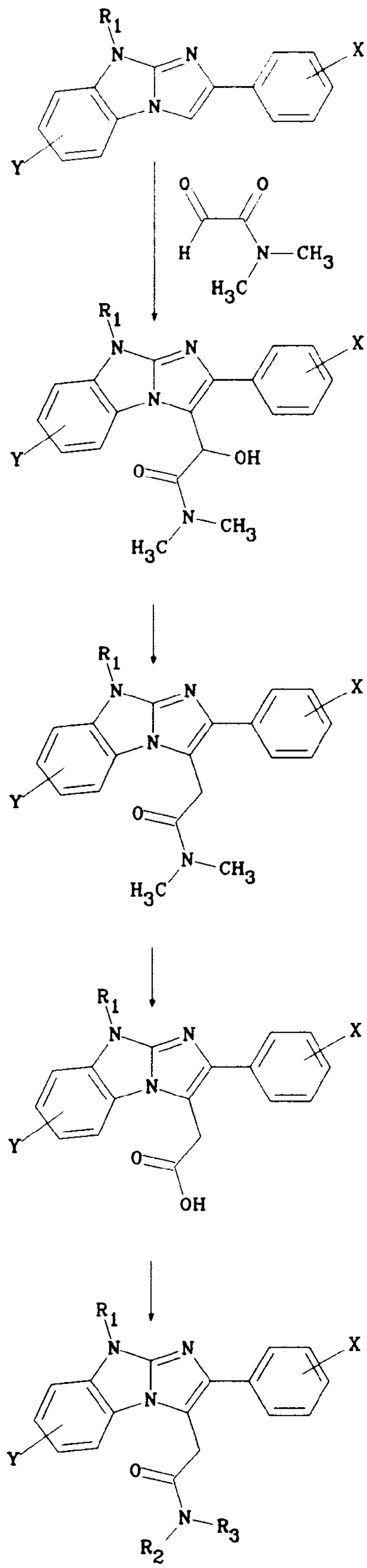

(II)

(III)

(Ia)

(IV)

(I)

solvant inerte miscible à l'eau, par exemple le dioxane ou le tétrahydrofurane, à une température de -40 à 40°C,

soit avec un agent réducteur tel qu'un hyposulfite ou un dithionite alcalin, par exemple l'hyposulfite ou le dithionite de sodium, ou encore avec l'hydroxyméthylsulfoxylate de sodium (Rongalite®), dans un solvant inerte, par exemple un solvant chloré tel que le dichlorométhane, éventuellement en présence d'un cosolvant inerte miscible à l'eau, par exemple le *N,N*- diméthylformamide ou la *N*-méthylpyrrolidone, à une température de 20 à 40°C.

On obtient ainsi un dérivé de *N,N*-diméthylacétamide de formule générale (Ia), qui correspond à la formule générale (I) lorsque $R_2$ et $R_3$ représentent chacun un groupe méthyle.

Si on désire préparer un composé de formule générale (I) dans laquelle $R_2$ et $R_3$ ne représentent pas chacun un groupe méthyle, on transforme le composé de formule générale (Ia) en acide de formule générale (IV), par hydrolyse au moyen d'une base forte, par exemple la soude ou la potasse, dans un solvant protique, par exemple l'éthanol ou le 2-méthoxyéthanol, en présence d'eau.

On fait ensuite réagir l'acide de formule générale (IV) avec le *N,N'*-carbonyldiimidazole, dans un solvant inerte, par exemple un solvant chloré ou éthéré tel que le dichlorométhane ou le tétrahydrofurane, à une température de 20 à 50°C, pour obtenir l'imidazolide correspondant, et finalement on traite ce dernier avec une amine de formule générale $HNR_2R_3$ (dans laquelle $R_2$ et $R_3$ sont tels que définis ci-dessus) à une température de 0 à 25°C.

Les composés de formules générales (III) et (IV) sont nouveaux et font partie de l'invention, à titre d'intermédiaires de synthèse du procédé illustré par le schéma qui précède.

Les dérivés de formule générale (II) peuvent être préparés selon toute méthode décrite dans la littérature, par exemple dans *J. Het. Chem.*, **2**, 287 (1965), *Khim. Geterosikl. Soedin.* 133 (1967), *J. Med. Chem.*, **15**, 923 (1972).

Les composés de l'invention peuvent également être préparés selon un autre procédé, illustré par le schéma 2 qui suit.

On fait réagir un dérivé de 9*H*-imidazo[1,2-*a*]benzimidazole de formule générale (II) (dans laquelle X, Y et $R_1$ sont tels que définis ci-dessus) avec le glyoxylate d'éthyle (que l'on prépare *in situ* au moyen de 2,2-diéthoxyacétate d'éthyle, comme décrit dans la demande de brevet EP-251859) dans un solvant protique tel que l'acide acétique à une température de 20 à 80°C.

On traite ensuite le dérivé d'α-hydroxyacétate d'éthyle de formule générale (V) avec un polyhalogénure d'acide sulfurique ou phosphorique, par exemple le chlorure de thionyle ou l'oxychlorure de phosphore, ou tout autre agent équivalent, dans un solvant inerte, par exemple un solvant chloré ou éthéré tel que le dichlorométhane ou le tétrahydrofurane, à une température de 20 à 80°C, pour former le dérivé de α-halogénoacétate d'éthyle correspondant, puis on fait réagir ce dernier soit avec un agent réducteur tel qu'un hydrure alcalin simple ou complexe, par exemple le borohydrure de sodium ou de potassium, dans un solvant protique, par exemple un alcool aliphatique tel que le méthanol ou l'éthanol, ou dans un solvant inerte miscible à l'eau, par exemple le dioxane ou le tétrahydrofurane, à une température de -40 à 40°C, soit avec un agent réducteur tel qu'un hyposulfite ou un dithionite alcalin, par exemple l'hyposulfite ou le dithionite de sodium, ou encore avec l'hydroxyméthylsulfoxylate de sodium (Rongalite®), dans un solvant inerte, par exemple un solvant chloré tel que le dichlorométhane, éventuellement en présence d'un cosolvant inerte miscible à l'eau, par exemple le *N,N*-diméthylformamide ou la *N*-méthyl-pyrrolidone, à une température de 20 à 40°C.

On obtient ainsi un composé de formule générale (VI), que l'on fait ensuite réagir avec un excès d'amine de formule générale $HNR_2R_3$ (dans laquelle $R_2$ et $R_3$ sont tels que définis ci-dessus) dans un solvant protique tel qu'un alcool, par exemple le méthanol ou l'éthanol, à une température de 0 à 70°C.

Schéma 2

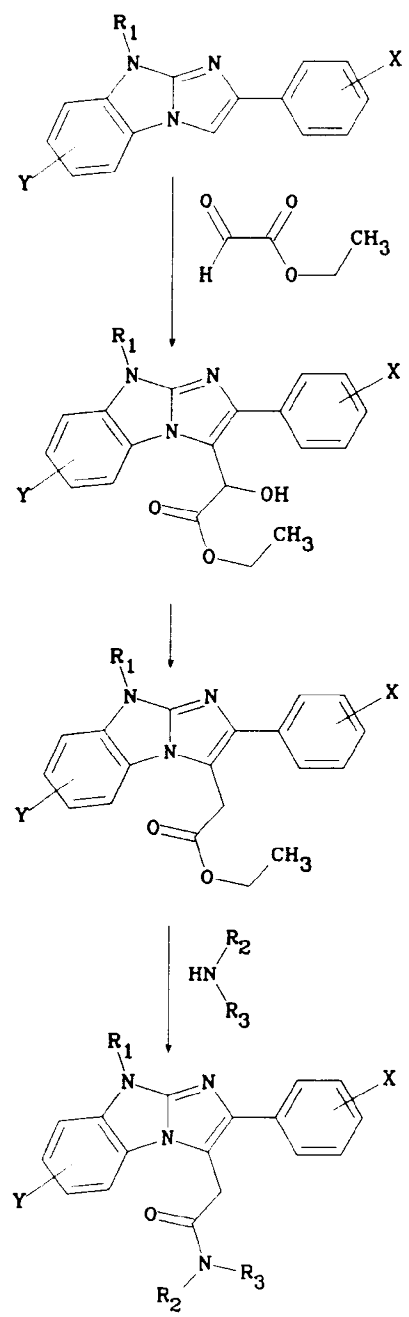

(II)

(V)

(VI)

(I)

Les composés de formule générale (VI) sont nouveaux et font partie de l'invention, à titre d'intermédiaires de synthèse du procédé illustré par le schéma 2.

Les exemples qui vont suivre illustrent la préparation de quelques composés de l'invention. Les microanalyses élémentaires, et les spectres I.R. et R.M.N. confirment les structures des composés obtenus.

Les numéros indiqués entre parenthèses dans les titres des exemples correspondent à ceux de la lère colonne du tableau 1 donné plus loin. Ce tableau illustre les structures chimiques et les propriétés physiques de quelques composés de formule générale (I).

Exemple 1 (Composé N°2)

*N,N*-diméthyl-2-phényl-9*H*-imidazo[1,2-*a*]benzimidazole-3-acétamide.

1.1. α-Hydroxy-*N,N*-diméthyl-2-phényl-9*H*-imidazo[1,2-*a*]benzimidazole-3-acétamide.

On mélange 13,5 g (0,077 mole) de 2,2-diéthoxy-*N,N*-diméthylacétamide et 1,7 ml (0,019 mole) d'acide chlorhydrique à 35% dans 125 ml d'acide acétique, et on chauffe en agitant dans un bain à 45°C pendant 1h. On ajoute ensuite 6,3 g (0,077 mole) d'acétate de sodium puis, après 15mn, 6 g (0,026 mole) de 2-phényl-9*H*-imidazo[1,2-*a*]benzimidazole, et on maintient le chauffage et l'agitation pendant 10h.
On évapore le mélange sous pression réduite et à température inférieure à 40°C, on reprend le résidu avec de l'eau et du dichlorométhane, on ajoute de l'ammoniaque jusqu'à pH 11, on élimine un insoluble par filtration, on sépare la phase organique, on la lave à l'eau, on la sèche sur sulfate de sodium, on évapore le solvant sous pression réduite, on fait cristalliser le résidu dans l'éther diéthylique, on l'essore, on le lave à l'éther diéthylique et on le sèche sous vide.
On obtient 6 g de composé.
Point de fusion : 215°C (décomposition).

1.2 *N,N*-diméthyl-2-phényl-9*H*-imidazo[1,2-*a*]benzimidazole-3-acétamide.

On traite 6 g (0,018 mole) de α-hydroxy-*N,N*-diméthyl-2-phényl-9*H*-imidazo[1,2-*a*]benzimidazole-3-acétamide avec 30 ml de chlorure de thionyle dans 300 ml de dichlorométhane à température ambiante pendant 24h.
On évapore le solvant et l'excès de chlorure de thionyle sous pression réduite, on fait cristalliser le résidu dans l'éther diéthylique, on l'essore et on le sèche.
On obtient 7,1 g de chlorhydrate de α-chloro-*N,N*-diméthyl-2-phényl-9*H*-imidazo[1,2-*a*]benzimidazole-3-acétamide que l'on réduit au moyen de 8,3 g (0,054 mole) de Rongalite® dans 300 ml de dichlorométhane à température ambiante pendant 24h. On reprend la suspension avec une solution aqueuse saturée de bicarbonate de sodium, on sépare la phase organique, on la sèche sur sulfate de sodium, on évapore le solvant sous pression réduite, on fait cristalliser le résidu dans l'éther diéthylique, on l'essore, on le lave à l'éther diéthylique, on le sèche et on le recristallise dans l'éthanol.
On obtient 2,35 g de composé.
Point de fusion : 268-271°C (décomposition).

<u>Exemple 2</u> (Composé N°39)

*N*,9-diméthyl-2-(4-méthylphényl)-9*H*-imidazo[1,2-*a*]benzimidazole-3-acétamide.

2.1 α-Hydroxy-*N,N*,9-triméthyl-2-(4-méthylphényl)-9*H*-imidazo[1,2-*a*]benzimidazole-3-acétamide.

On dissout 38,2 g (0,22 mole) de 2,2-diéthoxy-*N,N*-diméthylacétamide et 4,85 ml (0,055 mole) d'acide chlorhydrique à 35% dans 350 ml d'acide acétique, et on chauffe le mélange en l'agitant dan un bain à 45°C pendant 1h.
On ajoute ensuite 17,9 g (0,22 mole) d'acétate de sodium et, après 15mn, 19 g (0,073 mole) de 9-méthyl-2-(4-méthylphényl)-9*H*-imidazo[1,2-*a*]benzimidazole, et on maintient le chauffage et l'agitation pendant 2h30.
On évapore le mélange sous pression réduite à température inférieure à 40°C, on reprend le résidu avec 200 ml de dichlorométhane et 200 ml d'eau glacée, on ajoute de l'ammoniaque jusqu'à pH 11, on élimine un insoluble par filtration, on sépare la phase organique, on la lave à l'eau, on la sèche sur sulfate de sodium, on évapore le solvant sous pression réduite, on fait cristalliser le résidu dans l'éther diéthylique, on l'essore, on le lave à l'éther diéthylique et on le sèche sous vide.
On obtient 21,8 g de composé.
Point de fusion : 192-193°C.

2.2 *N,N*,9-triméthyl-2-(4-méthylphényl)-9*H*-imidazo[1,2-*a*]benzimidazole-3-acétamide.

On dissout 19,2 g (0,053 mole) de α-hydroxy-*N,N*,9-triméthyl- 2-(4-méthylphényl)-9*H*-imidazo[1,2-*a*] benzimidazole-3-acétamide dans 960 ml de dichlorométhane et on traite la solution avec 96 ml de chlorure de thionyle à température ambiante pendant 24h.
On évapore le solvant et l'excès de chlorure de thionyle sous pression réduite, on fait cristalliser le résidu dans l'éther diéthylique, on essore le solide obtenu, on le lave à l'éther diéthylique, et on le sèche rapidement à l'air.
On obtient 21,9 de chlorhydrate de α-chloro-*N,N*,9-triméthyl-2-(4-méthylphényl)-9*H*-imidazo[1,2-*a*]benzimidazole-3-acétamide que l'on réduit au moyen de 31,1 g (0,154 mole) de Rongalite® dans 960 ml de dichlorométhane à température ambiante pendant 36h.
On lave le mélange avec une solution aqueuse à 4% de bicarbonate de sodium, puis avec de l'eau, on sèche la phase organique sur sulfate de sodium, on évapore le solvant sous pression réduite, on reprend le résidu avec de l'éther diéthylique, on sépare le solide par filtration, on le lave avec de l'éther diéthylique et on le sèche sous vide.
On obtient 14,1 g de composé.
Point de fusion : 195-196°C.

2.3 Acide 9-méthyl-2-(4-méthylphényl)-9*H*-imidazo[1,2-*a*] benzimidazole-3-acétique.

On dissout 11 g (31,7 mmoles) de *N,N*,9-triméthyl-2-(4-méthylphényl)-9*H*-imidazo[1,2-*a*]benzimidazole-3-acétamide dans 330 ml de 2-méthoxyéthanol, on ajoute une solution de 6,35 g (0,159 mole) de soude en pastilles dans 44 ml d'eau, et on chauffe le mélange au reflux pendant 10h.
On évapore le solvant sous pression réduite, on reprend le résidu avec de l'eau, on élimine un léger insoluble par filtration, et on ajuste le pH du filtrat à 5 au moyen d'acide acétique.
On obtient un précipité qu'on sépare par filtration, on le lave à l'eau, on le sèche sous vide, on le reprend avec de l'éthanol, on le filtre, on le lave à l'éthanol et on le sèche sous vide.
On obtient 6,7 g de composé.
Point de fusion : 250-254°C (décomposition).

2.4 *N*,9-diméthyl-2-(4-méthylphényl)-9*H*-imidazo[1,2-*a*]benzimidazole-3-acétamide.

On prépare une suspension de 1,9 g (0,006 mole) d'acide 9-méthyl-2-(4-méthylphényl)-9*H*-imidazo[1,2-*a*]benzimidazole- 3-acétique dans 50 ml de tétrahydrofurane sec, on ajoute, par petites portions, 1,45 g (0,009 mole) de *N,N'*-carbonyldiimidazole sous atmosphère inerte, et on agite le mélange à température ambiante pendant 2h.
On refroidit le mélange dans un bain d'eau glacée, on le traite avec un excès de méthylamine sèche, et on maintient l'agitation pendant 16h à température ambiante.
On évapore le solvant sous pression réduite, on reprend le résidu avec de l'eau, on le sépare par filtration, on le lave à l'eau, on le sèche sous vide et on le purifie par recristallisation dans le méthanol.
On obtient 1 g de composé.
Point de fusion : 251-252°C.

<u>Exemple 3</u> (Composé N°57)

9-Ethyl-2-(4-méthylphényl)-9*H*-imidazo[1,2-*a*]benzimidazole-3-acétamide.

3.1 9-Ethyl-α-hydroxy-*N,N*-diméthyl-2-(4-méthylphényl)-9*H*-imidazo[1,2-*a*]benzimidazole-3-acétamide.

On dissout 27 g (0,145 mole) de 2,2-diéthoxy-*N,N*-diméthylacétamide et 4 ml (0,038 mole) d'acide chlorhydrique à 35% dans 150 ml d'acide acétique, et on chauffe le mélange en l'agitant dans un bain à 40°C pendant 1h.
On ajoute ensuite 12,7 g (0,145 mole) d'acétate de sodium puis, après 15mn, 14 g (0,05 mole) de 9-éthyl-2-(4-méthylphényl)-9*H*-imidazo[1,2-a]benzimidazole en solution dans 100 ml d'acide acétique, et on maintient le chauffage et l'agitation pendant 3h.
On évapore le solvant sous pression réduite et à température inférieure à 40°C, on reprend le résidu avec de l'eau et du dichlorométhane, on ajuste le pH à 11 avec de l'ammoniaque, on élimine un insoluble par filtration, on sépare la phase organique, on la lave à l'eau, on la sèche sur sulfate de sodium, on évapore le solvant sous pression réduite, on fait cristalliser le résidu dans l'éther diéthylique, on l'essore, on le lave et on le sèche sous vide.
On obtient 14,2 g de composé qu'on utilise tel quel dans l'étape suivante.

3.2 9-Ethyl-*N,N*-diméthyl-2-(4-méthylphényl)-9*H*-imidazo-[1,2-*a*]benzimidazole-3-acétamide.

On traite 14 g (0,037 mole) de 9-éthyl-α-hydroxy-*N,N*-diméthyl-2-(4-méthylphényl)-9*H*-imidazo[1,2-*a*]benzimidazole-3-acétamide avec 50 ml de chlorure de thionyle dans 500 ml de dichlorométhane à température ambiante pendant 24h.
On évapore le solvant et l'excès de chlorure de thionyle sous pression réduite, on fait cristalliser le résidu dans

l'éther diéthylique, on le sèche rapidement et on le dissout dans 500 ml de dichlorométhane. On ajoute 24 g (0,156 mole) de Rongalite® et on agite le mélange à température ambiante pendant 24h.
On élimine l'insoluble par filtration, on lave le filtrat avec une solution aqueuse de bicarbonate de sodium puis avec de l'eau, on le sèche sur sulfate de sodium, on évapore le solvant sous pression réduite et on purifie le résidu par chromatographie sur colonne de gel de silice en éluant avec un mélange 97,5/2,5 de dichlorométhane/méthanol.
On obtient 4,5 g de composé sous forme de base.
Point de fusion : 90°C.
On en prépare le chlorhydrate au moyen d'une solution d'acide chlorhyrique 0,1N dans le propan-2-ol.
Point de fusion : 247-251°C (décomposition).

3.3 Acide 9-éthyl-2-(4-méthylphényl)-9*H*-imidazo[1,2-*a*]benzimidazole-3-acétique.

On dissout 3,45 g (0,0096 mole) de 9-éthyl-*N,N*-diméthyl-2-(4-méthylphényl)-9*H*-imidazo[1,2-*a*]benzimidazole-3-acétamide dans 100 ml de 2-méthoxyéthanol, on ajoute une solution de 2,2 g (0,055 mole) de soude dans 15 ml d'eau, et on chauffe le mélange au reflux pendant 13h.
On évapore le solvant sous pression réduite, on reprend le résidu avec de l'eau, on élimine un insoluble par filtration, on ajoute de l'acide acétique jusqu'à pH 5, on sépare le précipité par filtration, on l'essore, on le lave et on le sèche.
On obtient 2,38 g de composé qu'on utilise tel quel dans l'étape suivante.
Point de fusion : 216-218°C (décomposition).

3.4 9-Ethyl-2-(4-méthylphényl)-9*H*-imidazo[1,2-*a*]benzimida- zole-3-acétamide.

On prépare une suspension de 1,1 g d'acide 9-éthyl-2-(4-méthylphényl)-9*H*-imidazo[1,2-*a*]benzimidazole-3-acétique dans 30 ml de tétrahydrofurane sec, on ajoute, par petites portions, 0,9 g (0,0056 mole) de *N,N'*-carbonyldiimidazole, et on agite le mélange à température ambiante pendant 2h.
On traite la solution obtenue avec un excès d'ammoniac sec, et on maintient l'agitation à température ambiante pendant 8h.
On évapore le solvant sous pression réduite, et on purifie le résidu par recrisatllisation dans un mélange de méthanol et de propan-2-ol.
On obtient 0,77 g de composé.
Point de fusion : 251-254°C (décomposition).

Exemple 4 (Composé N°11).

*N,N*,6,9-Tétraméthyl-2-phényl-9*H*-imidazo[1,2-*a*]benzimidazole-3-acétamide.

4.1. α-Hydroxy-*N,N*,6,9-tétraméthyl-2-phényl-9*H*-imidazo-[1,2-*a*]benzimidazole-3-acétamide.

On dissout 28,03 g (0,16 mole) de 2,2-diéthoxy-*N,N*-diméthylacétamide et 3,6 ml (0,0432 mole) d'acide chlorhydrique concentré dans 130 ml d'acide acétique, et on chauffe le mélange pendant 1h à 40°C.
On ajoute ensuite 13,1 g (0,16 mole) d'acétate de sodium, on agite pendant 15mn puis on ajoute une solution de 13,81 g (0,0528 mole) de 6,9-diméthyl-2-phényl-9*H*-imidazo[1,2-*a*]benzimidazole dans 70 ml d'acide acétique et on maintient le chauffage à 40-45°C pendant 3h. On évapore le solvant sous pression réduite et à une température inférieure à 40°C, on reprend le résidu par 200 ml de dichlorométhane et 200 ml d'eau glacée et on ajuste le pH à 10-11 au moyen d'hydroxyde de sodium. On élimine l'insoluble par filtration, on sépare la phase organique par décantation, on la lave à l'eau, on la sèche sur sulfate de sodium et on évapore le solvant sous pression réduite. On sèche le résidu sous pression réduite en présence de pentoxyde de phosphore. On obtient 12,73 g de solide que l'on utilise tel quel dans l'étape suivante.

4.2. *N,N*,6,9-Tétraméthyl-2-phényl-9*H*-imidazo[1,2-*a*]benzimidazole-3-acétamide.

On traite 12,73 g (0,035 mole) d'α-hydroxy-*N,N*,6,9-tétraméthyl-2-phényl-9*H*-imidazo[1,2-*a*]benzimidazole-3-acétamide avec 75 ml de chlorure de thionyle dans 250 ml de dichlorométhane pendant 24h. On évapore le solvant et l'excès de chlorure de thionyle sous pression réduite, on reprend le résidu avec du toluène et on évapore à nouveau. On reprend le résidu par 600 ml de dichlorométhane, on ajoute 22 g de Rongalite® et on agite le mélange pendant 48h.

On élimine l'insoluble par filtration, on lave le filtrat à l'eau et avec une solution aqueuse de bicarbonate de soude et à nouveau à l'eau. On sèche la phase organique sur sulfate de sodium, on évapore le solvant sous pression réduite et on isole 10,36 g de solide que l'on purifie par chromatographie sur colonne de gel de silice en éluant avec un mélange de chloroforme/méthanol 90/10. On isole 7,86 g de produit dont on prélève 1,3 g que l'on dissout dans du dichlorométhane, on filtre la solution et on évapore le filtrat sous pression réduite. On traite le résidu avec du noir de charbon dans du méthanol, on filtre la suspension, on évapore le méthanol sous pression réduite et on sèche le résidu sous vide. On obtient 0,9 g de solide blanc.
Point de fusion : 222-223°C.

Exemple 5 (Composé N°10).

*N*,6,9-Triméthyl-2-phényl-9*H*-imidazo[1,2-*a*]benzimidazole-3-acétamide.

5.1. Acide 6,9-diméthyl-2-phényl-9*H*-imidazo[1,2-*a*]benzimidazole-3-acétique.

On dissout 5,2 g (0,015 mole) de *N,N*,6,9-tétraméthyl-2-phényl-9*H*-imidazo[1,2-*a*]benzimidazole-3-acétamide dans 130 ml de 2-méthoxyméthanol, on ajoute une solution de 3,5 g (0,0875 mole) d'hydroxyde de sodium dans 20 ml d'eau et on chauffe le mélange au reflux pendant 3h30. On évapore le solvant sous pression réduite, on reprend le résidu avec 150 ml d'eau et on ajuste le pH jusqu'à 5 avec 10 ml d'acide acétique. On collecte l'insoluble par filtration, on le met en solution dans 100 ml de 2-méthoxyméthanol en présence de 4 g d'hydroxyde de potassium et 20 ml d'eau, et on chauffe à nouveau pendant 3h30.
On évapore le solvant sous pression réduite, on reprend le résidu par de l'eau et on ajoute de l'acide acétique jusqu'à pH = 5. On collecte l'insoluble par filtration, on le lave à l'eau et on le sèche. On obtient 4,51 g de solide qu'on utilise tel quel dans l'étape suivante.

5.2. *N*,6,9-triméthyl-2-phényl-9*H*-imidazo[1,2-*a*]benzimidazole-3-acétamide.

On prépare une suspension de 1,5 g (0,0047 mole) d'acide 6,9-diméthyl-2-phényl-9*H*-imidazo[1,2-*a*]benzimidazole-3-acétique dans 50 ml de tétrahydrofurane sec, on y ajoute 1,22 g (0,0075 mole) de *N,N'*-carbonyldiimidazole et on agite le mélange pendant 2h.
On traite la solution obtenue avec un excès de méthylamine gazeuze sèche et on agite pendant plusieurs heures.
On évapore le solvant sous pression réduite, on reprend le résidu par de l'eau et on isole un solide par filtration. Après séchage on purifie le produit par chromatographie sur colonne de gel de silice en éluant avec un mélange de dichlorométhane/méthanol 95/5, puis on traite le produit obtenu avec une solution d'acide chlorhydrique, on collecte le précipité par filtration et on le traite avec un excès d'ammoniaque jusqu'à un pH de 9 à 10. On isole un solide que l'on lave à l'eau et que l'on sèche. On obtient 0,79 g de produit.
Point de fusion : 251-253°C.

Exemple 6 (Composé N°9).

6,9-Diméthyl-2-phényl-9*H*-imidazo[1,2-*a*]benzimidazole-3-acétamide.

On prépare une suspension de 1,5 g (0,0047 mole) d'acide 6,9-diméthyl-2-phényl-9*H*-imidazo[1,2-*a*]benzimidazole-3-acétique dans 50 ml de tétrahydrofurane sec. On y ajoute 1,22 g (0,0075 mole) de *N,N'*-carbonyldiimidazole et on agite pendant 2h à 40-45°C, puis on traite la solution obtenue avec un excès d'ammoniac sec et on agite pendant 2h.
On évapore le solvant sous pression réduite, on lave le résidu à l'eau puis on le sèche. On purifie le produit obtenu par chromatographie sur colonne de gel de silice en éluant avec un mélange de chloroforme/acétone 95/5 et on isole 0,71 g de produit.
Point de fusion : 247-249°C (décomposition).

Exemple 7 (Composé N°17).

6-Chloro-*N,N*,9-triméthyl-2-phényl-9*H*-imidazo[1,2-*a*]benzimidazole-3-acétamide.

7.1. 6-Chloro-α-hydroxy-*N,N*,9-triméthyl-2-phényl-9*H*-imidazo[1,2-*a*]benzimidazole-3-acétamide.

On dissout 26,3 g (0,148 mole) de 2,2-diéthoxy-*N,N*-diméthylacétamide et 3,5 ml (0,042 mole) d'acide chlorhydrique concentré dans 130 ml d'acide acétique et on chauffe le mélange pendant 1h à 40°C. On ajoute ensuite 12,3 g (0,15 mole) d'acétate de sodium, on agite 15mn puis on ajoute une solution de 14,19 g (0,05 mole) de 6-chloro-9-méthyl-2-phényl-9*H*-imidazo[1,2-*a*]benzidimazole dans 70 ml d'acide acétique. On agite le mélange pendant 3h à 40°C, puis on évapore le solvant sous pression réduite à une température inférieure à 40°C. On reprend le résidu par 200 ml d'eau glacée et 200 ml de dichlorométhane, on agite 5mn, puis on traite avec un excès d'ammoniaque jusqu'à pH basique. On élimine un insoluble par filtration, on décante la phase organique, on la lave à l'eau et on la sèche sur sulfate de sodium. On évapore le solvant sous pression réduite et on obtient 16,5 g de solide que l'on utilise tel quel dans l'étape suivante.

7.2 6-Chloro-*N,N*,9-triméthyl-2-phényl-9*H*-imidazo[1,2-*a*]-benzimidazole-3-acétamide.

On traite 16,5 g (0,043 mole) de 6-chloro-α-hydroxy-*N,N*,9-triméthyl-2-phényl-9*H*-imidazo[1,2-*a*]benzimidazole-3-acétamide avec 90 ml de chlorure de thionyle dans 250 ml de dichlorométhane, on agite pendant 6h et on laisse au repos pendant une nuit. On évapore le solvant et l'excès de chlorure de thionyle sous pression réduite, on reprend le résidu avec du toluène et on évapore à nouveau.
On dissout le résidu dans 800 ml de dichlorométhane et on le traite par 26,5 g de Rongalite®. On agite le mélange pendant 48h puis on sépare l'insoluble par filtration et on lave la phase organique au bicarbonate de soude puis à l'eau. On évapore le solvant sous pression réduite et on reprend le résidu avec du pentane. On obtient 14,96 g de solide que l'on purifie par chromatographie sur colonne de gel de silice en éluant avec un mélange de dichlorométhane/méthanol 95/5. On isole 8,59 g de produit dont on prend un échantillon de 1,4 g que l'on dissout dans du dichlorométhane, on filtre la solution et on concentre le filtrat sous pression réduite. On traite le résidu avec du noir de charbon dans le méthanol, puis on recristallise le produit dans un mélange de *N,N*- diméthylformamide/eau 2/1 et on obtient 0,98 g de solide.
Point de fusion : 233-236°C.

Exemple 8 (Composé N°16).

6-Chloro-*N*,9-diméthyl-2-phényl-9*H*-imidazo[1,2-*a*]benzimidazole-3-acétamide.

8.1. Acide 6-chloro-9-méthyl-2-phényl-9*H*-imidazo[1,2-*a*]benzimidazole-3-acétique.

On dissout 7 g (0,019 mole) de 6-chloro-*N,N*,9-triméthyl-2-phényl-9*H*-imidazo[1,2-*a*]benzimidazole-3-acétamide dans 150 ml de 2-méthoxyéthanol, on ajoute 4,5 g (0,112 mole) d'hydroxyde de sodium en solution dans 25 ml d'eau et on chauffe le mélange pendant 3h au reflux. On évapore le solvant sous pression réduite, on reprend le résidu avec de l'eau et on acidifie la solution obtenue par de l'acide acétique. On collecte le solide par filtration, on le dissout dans un mélange de 100 ml de 2-méthoxyéthanol, 20 ml d'eau et 5 g (0,089 mole) d'hydroxyde de potassium et on agite et chauffe au reflux pendant 8h30. On évapore le solvant sous pression réduite, on reprend le résidu à l'eau et acidifie à pH = 5 avec de l'acide acétique. On isole l'insoluble par filtration et le sèche. On obtient 5,76 g de solide.
Point de fusion : 245-250°C (décomposition).

8.2. 6-Chloro-*N*,9-diméthyl-2-phényl-9*H*-imidazo[1,2-*a*]benzimidazole-3-acétamide.

On prépare une suspension de 1,5 g (0,044 mole) d'acide 6-chloro-9-méthyl-2-phényl-9*H*-imidazo[1,2-*a*]benzimidazole-3-acétique dans 50 ml de tétrahydrofurane sec, on y ajoute 1,15 g (0,071 mole) de *N,N'*-carbonyldiimidazole et agite pendant 18h. On traite ensuite la solution par un excès de méthylamine gazeuse sèche et agite encore pendant 6h. On évapore le solvant sous pression réduite, on reprend le résidu à l'eau et au bicarbonate de soude puis à l'eau à nouveau, on sépare l'insoluble par filtration et on le sèche. On le purifie par chromatographie sur colonne de gel de silice en éluant avec un mélange de dichlorométhane/méthanol 97/3. On traite le produit obtenu par une solution d'acide chlorhydrique, on collecte le précipité par filtration et on le traite avec un excès d'ammoniaque. On recristallise le produit obtenu dans un mélange *N,N*-diméthylformamide/eau 75/25 puis on le sèche à 100°C sous vide. On obtient 0,8 g de solide.
Point de fusion : 272-273,5°C (décomposition) .

Exemple 9 (Composé N°15)

6-Chloro-9-méthyl-2-phényl-9*H*-imidazo[1,2-*a*]benzimidazole-3-acétamide.

On procède de la même manière que celle décrite pour l'exemple 6 en partant de 1,5 (0,044 mole) d'acide 6-chloro-9-méthyl-2-phényl-9*H*-imidazo[1,2-*a*]benzimidazole-3-acétique, et on obtient, après purification, 0,9 g d'amide.
Point de fusion : 253-254°C (décomposition).

Exemple 10 (Composé N°20)

7-Chloro-*N,N,*9-triméthyl-2-phényl-9*H*-imidazo[1,2-*a*]benzimidazole-3-acétamide.

10.1. 7-Chloro-α-hydroxy-*N,N,*9-triméthyl-2-phényl-9*H*-imidazo[1,2-*a*]benzimidazole-3-acétamide.

On dissout 42,05 g (0,24 mole) de 2,2-diéthoxy-*N,N*-diméthylacétamide et 5,6 ml (0,067 mole) d'acide chlorhydrique concentré dans 150 ml d'acide acétique, et on agite le mélange à 40°C pendant 1h. On y ajoute ensuite 19,65 g (0,24 mole) d'acétate de sodium, on agite pendant 15 à 20mn, puis on ajoute une suspension de 22,45 g (0,08 mole) de 7-chloro-6-méthyl-2-phényl-9*H*-imidazo[1,2-*a*]benzimidazole dans 150 ml d'acide acétique. On agite le mélange pendant 4h à 40-50°C, on évapore le solvant sous pression réduite à une température inférieure à 45°C, on reprend le résidu par 300 ml d'eau glacée et 250 ml de dichlorométhane, on alcalinise au moyen de soude, on sépare l'insoluble par filtration, on le lave au dichlorométhane. On décante le filtrat, on sèche la phase organique sur sulfate de sodium, et on évapore le solvant sous pression réduite. On obtient 25 g de produit que l'on utilise tel quel dans l'étape suivante.

10.2. 7-Chloro-*N,N*,9-triméthyl-2-phényl-9*H*-imidazo[1,2-*a*]benzimidazole-3-acétamide.

On traite 21,05 g (0,0548 mole) de 7-chloro-α-hydroxy-*N,N*,9-triméthyl-2-phényl-9*H*-imidazo[1,2-*a*]benzimidazole-3-acétamide avec 115 ml de chlorure de thionyle et on agite le mélange pendant 9h. On évapore l'excès de chlorure de thionyle sous pression réduite, on reprend le résidu avec du toluène et on évapore ce dernier. On dissout le résidu dans 800 ml de dichlorométhane, on ajoute 34 g de Rongalite® et on agite le mélange pendant 48h. On élimine l'insoluble par filtration, on lave le filtrat au bicarbonate de soude puis à l'eau, on sèche la phase organique sur sulfate de sodium puis on la concentre. On purifie le résidu par chromatographie sur colonne de gel de silice en éluant avec un mélange chloroforme/acétone 95/5. On obtient 7,25 g de composé dont on prélève un échantillon de 1,5 g afin de le recristalliser dans un mélange de *N,N*-diméthylformamide/eau 60/40. Après séchage, on obtient 1,36 g de produit.
Point de fusion : 192,5-195°C.

Exemple 11 (Composé N°19).

7-Chloro-*N*,9-diméthyl-2-phényl-9*H*-imidazo[1,2-*a*]benzimidazole-3-acétamide.

11.1. Acide 7-chloro-9-méthyl-2-phényl-9*H*-imidazo[1,2-*a*]benzimidazole-3-acétique.

On dissout 5,61 g (0,0145 mole) de 7-chloro-*N,N,*9-triméthyl-2-phényl-9*H*-imidazo[1,2-*a*]benzimidazole-3-acétamide dans 130 ml de 2-méthoxyméthanol, on y ajoute 4,7 g d'hydroxyde de sodium et 30 ml d'eau, puis on agite et chauffe le mélange au reflux pendant 4h. On évapore le solvant sous pression réduite, on reprend le résidu à l'eau et à l'acide acétique jusqu'à pH = 5. On met le précipité formé en suspension dans 100 ml de 2-méthoxyméthanol avec 4 g d'hydroxyde de potassium et 30 ml d'eau. On chauffe à nouveau au reflux pendant 8h, on évapore ensuite le solvant sous pression réduite, on reprend le résidu par de l'eau et acidifie jusqu'à pH = 5 la solution au moyen d'acide acétique. On isole un précipité que l'on sèche sous vide ; on obtient 4,52 g de produit.
Point de fusion : 230-232°C.

11.2. 7-Chloro-*N*,9-diméthyl-2-phényl-9*H*-imidazo[1,2-*a*]benzimidazole-3-acétamide.

On prépare une suspension de 1,7 g (0,005 mole) d'acide 7-chloro-9-méthyl-2-phényl-9*H*-imidazo[1,2-*a*]benzimidazole-3-acétique dans 50 ml de tétrahydrofurane sec, on y ajoute 1,3 g (0,008 mole) de *N,N'*-carbonyldiimidazole et on agite à 40°C pendant 2h.
On traite la solution obtenue par un excès de méthylamine gazeuse sèche et on l'agite pendant 72h. On évapore le solvant sous pression réduite, on traite le résidu par 50 ml d'eau, on sépare l'insoluble par filtration, on le lave à l'eau puis on le sèche.
On obtient 1,66 g de solide que l'on purifie par chromatographie sur gel de silice en éluant avec un mélange dichlorométhane/méthanol 95/5, on recristallise le produit dans un mélange *N,N*-diméthylformamide/eau 75/25 puis on le sèche. On obtient 1,27 g de solide.
Point de fusion : 274-275°C.

Exemple 12 (Composé N°28).

*N,N*,6,7,9-pentaméthyl-2-phényl-9*H*-imidazo[1,2-*a*]benzimidazole-3-acétamide.

12.1. α-hydroxy-*N,N*,6,7,9-pentaméthyl-2-phényl-9*H*-imidazo[1,2-*a*]benzimidazole-3-acétamide.

On dissout 22,6 g (0,128 mole) de 2,2-diéthoxy-*N,N*-diméthylacétamide et 3,4 ml (0,041 mole) d'acide chlorhydrique concentré dans 120 ml d'acide acétique, on agite pendant 1h à 40°C, puis on ajoute 10,7 g d'acétate de sodium et agite à nouveau pendant 15mn à 40-45°C. Ensuite on ajoute 11,86 g (0,043 mole) de *N*,6,7-triméthyl-2-phényl-9*H*-imidazo[1,2-*a*]-benzimidazole dissous dans 60 ml d'acide acétique et on agite le mélange pendant 2h à 40-45°C.
On évapore le solvant sous pression réduite à une température inférieure à 45°C, on reprend le résidu par de l'eau glacée et du dichlorométhane, on agite 5mn puis on basifie le mélange avec de l'ammoniaque. On isole un précipité par filtration, on lave le filtrat à l'eau, on sèche la phase organique sur sulfate de sodium puis on évapore le solvant sous pression réduite. On isole 7,09 g de produit. On reprend le précipité mentionné ci-dessus avec du méthanol, on filtre la suspension et on concentre le filtrat sous pression réduite : on obtient un second lot de produit de 5,72 g. On réunit les deux et on les utilise tels quels dans l'étape suivante.

12.2. *N,N*,6,7,9-Pentaméthyl-2-phényl-9*H*-imidazo[1,2-*a*]benzimidazole-3-acétamide.

On dissout 13,7 g (0,0363 mole) α-hydroxy-*N,N*,6,7,9-pentaméthyl-2-phényl-9*H*-imidazo[1,2-*a*]benzimidazole-3-acétamide dans 500 ml de dichlorométhane ; il se forme un léger insoluble que l'on élimine par filtration. Au filtrat on ajoute 75 ml de chlorure de thionyle et on agite le mélange pendant 3h, on le laisse au repos pendant une nuit et on l'agite à nouveau pendant 6h.
On évapore le solvant sous pression réduite, ainsi que l'excès de chlorure de thionyle au moyen de toluène.
On dissout le résidu dans 500 ml de dichlorométhane et on ajoute à cette solution 23,5 g de Rongalite®, on agite pendant 48h, on élimine l'insoluble par filtration, on lave la phase organique à l'eau, au bicarbonate de soude puis à l'eau, on la sèche sur sulfate de sodium et on la concentre sous pression réduite. On obtient 12,23 g de produit que l'on lave à l'éther puis que l'on purifie par chromatographie sur colonne de gel de silice en éluant avec un mélange de chloroforme/-acétone 95/5. On obtient 6,16 g de produit dont on prélève 1,32 g pour deux traitements successifs au noir de charbon dans le méthanol. On isole finalement 1,04 g de produit.
Point de fusion : 202-206°C.

Exemple 13 (Composé N°83).

2-(4-Chlorophényl)-*N*,6,9-triméthyl-9*H*-imidazo[1,2-*a*]benzimidazole-3-acétamide.

13.1. 2-(4-Chlorophényl)-α-hydroxy-*N,N*,6,9-tétraméthyl-9*H*-imidazo[1,2-*a*]benzimidazole-3-acétamide.

On dissout 25,6 g (0,146 mole) de 2,2-diéthoxy-*N,N*-diméthylacétamide et 4 ml (0,048 mole) d'acide chlorhydrique concentré dans 100 ml d'acide acétique et on chauffe le mélange à 40°C pendant 1h.
On ajoute 12 g (0,146 mole) d'acétate de sodium, on agite le mélange pendant 15mn, on ajoute une solution de 14,4 g (0,049 mole) de 2-(4-chlorophényl)-6,9-diméthyl-9*H*-imidazo[1,2-*a*]-benzimidazole dans 100 ml d'acide acétique, et on agite le mélange à 40°C pendant 5h.
On évapore le solvant sous pression réduite, a une température inférieure à 45°C, on reprend le résidu avec 300 ml d'eau glacée et 300 ml de chloroforme, on ajoute de la soude, on agite pendant 5mn, on élimine un insoluble par filtration, on sépare la phase organique, on la sèche sur sulfate de sodium, on évapore le solvant sous pression réduite, on reprend le résidu avec du pentane, on sépare l'insoluble par filtration, on l'essore, on le lave et on le sèche. On obtient 10,4 g de composé.

Point de fusion : 150°C.

13.2. 2-(4-Chlorophényl)-*N,N*,6,9-tétraméthyl-9*H*-imidazo[1,2-*a*]benzimidazole-3-acétamide.

On traite 10,2 g (0,026 mole) de 2-(4-chlorophényl)-α-hydroxy-*N,N*,6,9-tétraméthyl-9*H*-imidazo[1,2-*a*]benzimidazole-3-acétamide avec 50 ml de chlorure de thionyle dans 200 ml de dichlorométhane, on agite le mélange pendant 3h et on le laisse au repos pendant une nuit.
On évapore le solvant et l'excès de chlorure de thionyle sous pression réduite, on reprend le résidu avec de l'éther de pétrole, on le filtre, on le lave et on le sèche, puis on le traite avec 16 g (0,103 mole) de Rongalite® dans 450 ml de dichlorométhane, en agitant le mélange pendant 24h.
On sépare l'insoluble par filtration, on lave la phase organique avec une solution saturée d'hydrogénocarbonate de sodium puis avec de l'eau, on la sèche sur sulfate de sodium, on la filtre, on évapore le solvant sous pression réduite, on reprend le résidu avec du pentane, on purifie le solide obtenu par chromatographie sur colonne de gel de silice en éluant avec un mélange de chloroforme/acétone 93/7.
On isole 3,3 g de solide dont on prélève 0,7 g qu'on traite avec de l'acide chlorhydrique aqueux, on agite le mélange, on essore le solide, on le lave à l'eau, on le reprend avec une solution aqueuse de carbonate de sodium et du chloroforme, on sépare la phase organique, on la lave à l'eau, on la sèche, on évapore le solvant sous pression réduite, on reprend le résidu avec de l'éther diéthylique, on l'essore et on le sèche sous vide.
On obtient 0,46 g de composé.
Point de fusion : 239,5-241,5°C.

13.3. Acide 2-(4-chlorophényl)-6,9-diméthyl-9*H*-imidazo-[1,2-*a*]benzimidazole-3-acétique.

On dissout 2,63 g (0,069 mole) de 2-(4-chlorophényl)-*N,N*,6,9-tétraméthyl-9*H*-imidazo[1,2-*a*]benzimidazole-3-acétamide dans 80 ml de 2-méthoxyéthanol, on ajoute 4,8 g (0,087 mole) d'hydroxyde de potassium en solution dans 15 ml d'eau, et on chauffe le mélange au reflux pendant 9h.
On évapore le solvant sous pression réduite, on reprend le résidu avec de l'eau, on amène le pH à 5 avec de l'acide acétique, on sépare l'insoluble par filtration et on le sèche. On obtient 2,7 g de solide.
Point de fusion : 265-270°C (décomposition).

13.4. 2-(4-Chlorophényl)-*N*,6,9-triméthyl-9*H*-imidazo[1,2-*a*]-benzimidazole-3-acétamide.

Sous courant d'azote on prépare une suspension de 1,0 g (0,0028 mole) d'acide 2-(4-chlorophényl)-6,9-diméthyl-9*H*-imidazo[1,2-*a*]benzimidazole-3-acétique dans 50 ml de tétrahydrofurane sec, on ajoute 1,0 g (0,0063 mole) de *N,N'*-carbonyl-diimidazole en solution dans 50 ml de tétrahydrofurane sec, et on agite le mélange pendant 6h.
On traite ensuite la solution avec un excès de méthylamine gazeuse sèche, et on l'agite encore pendant 4h.
On évapore le solvant sous pression réduite, on reprend le résidu avec de l'eau, on l'essore, on le lave à l'eau et on le sèche. On obtient 0,65 g de produit qu'on purifie par chromatographie sur colonne de gel de silice en éluant avec un mélange de chloroforme/acétone 93/7.
On isole finalement 0,26 g de composé.
Point de fusion : 258-260°C.

Exemple 14 (Composé N°30).

*N*-Méthyl-2-(4-méthylphényl)-9*H*-imidazo[1,2-*a*]benzimidazole-3-acétamide.

14.1. α-Hydroxy-2-(4-méthylphényl)-9*H*-imidazo[1,2-*a*]benzimidazole-3-acétate d'éthyle.

On dissout 9,55 ml (0,053 mole) de 2,2-diéthoxyacétate d'éthyle et 1,2 ml (0,013 mole) d'acide chlorhydrique concentré dans 130 ml d'acide acétique, et on chauffe le mélange à 60°C pendant 30mn.
On ajoute 4,4 g (0,053 mole) d'acétate de sodium, on agite pendant 15mn, puis on ajoute, par petites portions, 6,6 g (0,027 mole) de 2-(4-méthylphényl)-9*H*-imidazo[1,2-*a*]benzimidazole, et on poursuit l'agitation à 60°C pendant 5h.
On évapore le solvant sous pression réduite, on reprend le résidu avec du dichlorométhane et de l'ammoniaque diluée, on sépare un insoluble par filtration, on sépare la phase organique, on la lave à l'eau jusqu'à pH neutre, on la sèche sur sulfate de sodium, on évapore le solvant sous pression réduite, on purifie le résidu par chromatographie sur colonne de gel de silice en éluant avec un mélange de dichlorométhane/méthanol 97/3. Après

recristallisation dans le pentane on obtient 6,5 g de produit rouge qu'on utilise tel quel dans l'étape suivante.

14.2. 2-(4-Méthylphényl)-9*H*-imidazo[1,2-*a*]benzimidazole-3-acétate d'éthyle.

On traite 6,3 g (0,018 mole) de α-hydroxy-2-(4-méthylphényl)-9*H*-imidazo[1,2-*a*]benzimidazole-3-acétate d'éthyle avec 18 ml de chlorure de thionyle dans 180 ml de dichlorométhane en agitant le mélange pendant 16h.
On évapore le solvant et l'excès de chlorure de thionyle sous pression réduite, on reprend le résidu avec de l'éther diéthylique, on le lave, on l'essore et on le sèche.
On le traite ensuite avec 7,8 g (0,051 mole) de Rongalite® dans 170 ml de dichlorométhane, en agitant le mélange à température ambiante pendant 16h.
On sépare le solide par filtration, on lave la phase organique avec une solution saturée d'hydrogénocarbonate de sodium puis avec de l'eau jusqu'à pH neutre, on la sèche sur sulfate de sodium, on évapore le solvant sous pression réduite, et on purifie le résidu par cristallisation dans l'éther diéthylique. On obtient 4,9 g de composé.
Point de fusion : 234-237°C (décomposition) .

14.3. *N*-Méthyl-2-(4-méthylphényl)-9*H*-imidazo[1,2-*a*]benzimidazole-3-acétamide.

On traite 2 g de 2-(4-méthylphényl)-9*H*-imidazo[1,2-*a*]benzimidazole-3-acétate d'éthyle dans 60 ml de méthanol avec 60 ml de méthylamine liquide, en agitant le mélange à température ambiante pendant 16h.
On évapore le solvant sous pression réduite, on lave le résidu à l'eau, et on le recristallise deux fois dans le *N,N*- diméthylformamide.
On isole finalement 0,8 g de composé.
Point de fusion : 303-308°C (décomposition) .

Exemple 15 (Composé N°42).

*N*-(2-méthoxyéthyl)-2-(4-méthylphényl)-9*H*-imidazo[1,2-*a*]benzimidazole-3-acétamide.

On prépare une suspension de 3,5 g (0,11 mole) d'acide 9-méthyl-2-(4-méthylphényl)-9*H*-imidazo[1,2-*a*]benzimidazole-3-acétique dans 70 ml de tétrahydrofurane sec, on ajoute 2,67 g (0,165 mole) de *N,N'*-carbonyldiimidazole par petites portions, en 10mn, et on agite le mélange à température ambiante pendant 1h.
On le refroidit dans un bain d'eau glacée, on ajoute 1,65 g (0,165 mole) de 2-méthoxyéthylamine en solution dans 4 ml de tétrahydrofurane sec, on poursuit l'agitation pendant 1h à froid, puis à température ambiante pendant 4h.
On évapore le solvant sous pression réduite, on fait cristalliser le résidu dans l'eau, on collecte le solide par filtration, on le lave, on le sèche, et on le recristallise dans l'éthanol.
On obtient finalement 2,9 g de composé.
Point de fusion : 189-190°C.

Le tableau 1 qui suit illustre les structures chimiques et les propriétés physiques de quelques composés de formule générale (I).
Dans la colonne "Sel", "-" désigne un composé à l'état de base, et "HCl" désigne un composé à l'état de chlorhydrate. Dans la colonne "F (°C)", "(d)" désigne une température de fusion avec décomposition.

Tableau 1

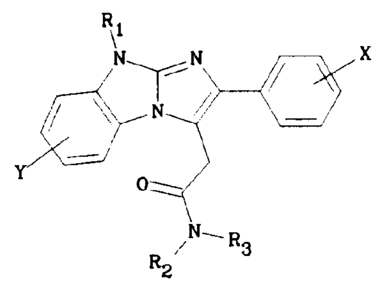

(I)

| N° | X | Y | $R_1$ | $R_2$ | $R_3$ | Sel | F (°C) |
|---|---|---|---|---|---|---|---|
| 1 | H | H | H | H | $CH_3$ | - | 316-321 (d) |
| 2 | H | H | H | $CH_3$ | $CH_3$ | - | 268-271 (d) |
| 3 | H | H | $CH_3$ | H | H | - | 238-239 |
| 4 | H | H | $CH_3$ | H | $CH_3$ | - | 253-254 (d) |
| 5 | H | H | $CH_3$ | $CH_3$ | $CH_3$ | - | 173-175 |
| 6 | H | H | $CO_2CH_2CH_3$ | $CH_3$ | $CH_3$ | - | 218-220 |
| 7 | H | 6-F | H | H | $CH_3$ | - | solide amorphe |
| 8 | H | 6-F | H | $CH_3$ | $CH_3$ | - | solide amorphe |
| 9 | H | 6-$CH_3$ | $CH_3$ | H | H | - | 247-249 (d) |
| 10 | H | 6-$CH_3$ | $CH_3$ | H | $CH_3$ | - | 251-253 |
| 11 | H | 6-$CH_3$ | $CH_3$ | $CH_3$ | $CH_3$ | - | 222-223 |

| N° | X | Y | $R_1$ | $R_2$ | $R_3$ | Sel | F (°C) |
|---|---|---|---|---|---|---|---|
| 12 | H | 6-Br | $CH_3$ | H | H | - | 268,5-270 |
| 13 | H | 6-Br | $CH_3$ | H | $CH_3$ | - | 272-280 (d) |
| 14 | H | 6-Br | $CH_3$ | $CH_3$ | $CH_3$ | - | 246,5-248 |
| 15 | H | 6-Cl | $CH_3$ | H | H | - | 253-254 (d) |
| 16 | H | 6-Cl | $CH_3$ | H | $CH_3$ | - | 272-273,5 (d) |
| 17 | H | 6-Cl | $CH_3$ | $CH_3$ | $CH_3$ | - | 233-236 |
| 18 | H | 7-Cl | $CH_3$ | H | H | - | 254,5-256,5 (d) |
| 19 | H | 7-Cl | $CH_3$ | H | $CH_3$ | - | 274-275 |
| 20 | H | 7-Cl | $CH_3$ | $CH_3$ | $CH_3$ | - | 192,5-195 |
| 21 | H | 6-F | $CH_3$ | H | H | - | 244-245 (d) |
| 22 | H | 6-F | $CH_3$ | H | $CH_3$ | - | 256,5-258 |
| 23 | H | 6-F | $CH_3$ | $CH_3$ | $CH_3$ | - | 201,5-202,5 |
| 24 | H | 6-$OCF_3$ | $CH_3$ | $CH_3$ | $CH_3$ | - | 139,5-141 |
| 25 | H | 6-$C(CH_3)_3$ | $CH_3$ | $CH_3$ | $CH_3$ | - | solide amorphe |
| 26 | H | 6,7-$(CH_3)_2$ | $CH_3$ | H | H | - | 238,5-239 (d) |
| 27 | H | 6,7-$(CH_3)_2$ | $CH_3$ | H | $CH_3$ | - | 275-276 (d) |
| 28 | H | 6,7-$(CH_3)_2$ | $CH_3$ | $CH_3$ | $CH_3$ | - | 202-206 |

| N° | X | Y | $R_1$ | $R_2$ | $R_3$ | Sel | F (°C) |
|---|---|---|---|---|---|---|---|
| 29 | H | 6,7-$Cl_2$ | $CH_3$ | $CH_3$ | $CH_3$ | - | 209-217 (d) |
| 30 | 4-$CH_3$ | H | H | H | $CH_3$ | - | 303-308 (d) |
| 31 | 4-$CH_3$ | H | H | $CH_3$ | $CH_3$ | - | 280-288 (d) |
| 32 | 2-$CH_3$ | H | $CH_3$ | H | H | - | 213-214,5 |
| 33 | 2-$CH_3$ | H | $CH_3$ | H | $CH_3$ | - | 213-215 |
| 34 | 2-$CH_3$ | H | $CH_3$ | $CH_3$ | $CH_3$ | - | 135-137 |
| 35 | 3-$CH_3$ | H | $CH_3$ | H | H | - | 235-238 |
| 36 | 3-$CH_3$ | H | $CH_3$ | H | $CH_3$ | - | 234-236 |
| 37 | 3-$CH_3$ | H | $CH_3$ | $CH_3$ | $CH_3$ | - | 178-179 |
| 38 | 4-$CH_3$ | H | $CH_3$ | H | H | - | 258-260 |
| 39 | 4-$CH_3$ | H | $CH_3$ | H | $CH_3$ | - | 251-252 |
| 40 | 4-$CH_3$ | H | $CH_3$ | H | $CH_2CH_3$ | - | 238-240 |
| 41 | 4-$CH_3$ | H | $CH_3$ | H | $CH_2CH_2CH_3$ | - | 237-239 |
| 42 | 4-$CH_3$ | H | $CH_3$ | H | $CH_2CH_2OCH_3$ | - | 189-190 |
| 43 | 4-$CH_3$ | H | $CH_3$ | H | $CH_2CH_2OC_6H_5$ | - | 220-221 |
| 44 | 4-$CH_3$ | H | $CH_3$ | H | 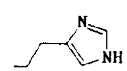 | - | 170-172 |

| N° | X | Y | $R_1$ | $R_2$ | $R_3$ | Sel | F (°C) |
|---|---|---|---|---|---|---|---|
| 45 | 4-$CH_3$ | H | $CH_3$ | H | 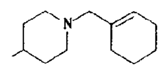 | - | 214-217 |
| 46 | 4-$CH_3$ | H | $CH_3$ | H | 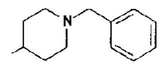 | - | 226-228 |
| 47 | 4-$CH_3$ | H | $CH_3$ | $CH_3$ | $CH_3$ | - | 195-196 |
| 48 | 4-$CH_3$ | H | $CH_3$ | H | 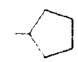 | - | 257-258,5 |
| 49 | 4-$CH_3$ | H | $CH_3$ | 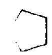 | | - | 190-192 |
| 50 | 4-$CH_3$ | H | $CH_3$ | 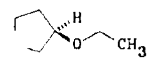 | | - | 164-166 |
| 51 | 4-$CH_3$ | H | $CH_3$ | 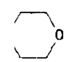 | | - | 230-231,5 |
| 52 | 4-$CH_3$ | H | $CH_3$ | 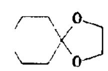 | | - | 195-196 |
| 53 | 4-$CH_3$ | H | $CH_3$ | 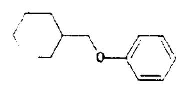 | | HCl | 234-236 |
| 54 | 4-$CH_3$ | H | $CH_3$ | 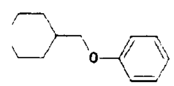 | | - | 199-201 |
| 55 | 4-$CH_3$ | H | $CH_3$ | 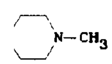 | | - | 222-224 |
| 56 | 4-$CH_3$ | H | $CH_3$ | 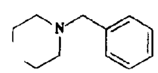 | | - | 162,5-163 |

| N° | X | Y | $R_1$ | $R_2$ | $R_3$ | Sel | F (°C) |
|---|---|---|---|---|---|---|---|
| 57 | 4-$CH_3$ | H | $CH_2CH_3$ | H | H | - | 251-254 (d) |
| 58 | 4-$CH_3$ | H | $CH_2CH_3$ | H | $CH_3$ | HCl | 235-238 (d) |
| 59 | 4-$CH_3$ | H | $CH_2CH_3$ | $CH_3$ | $CH_3$ | HCl | 247-251 (d) |
| 60 | 4-$CH_3$ | H | $CH_2C_6H_5$ | H | H | - | 279-284 (d) |
| 61 | 4-$CH_3$ | H | $CH_2C_6H_5$ | H | $CH_3$ | - | 222-225 |
| 62 | 4-$CH_3$ | H | $CH_2C_6H_5$ | $CH_3$ | $CH_3$ | - | 189-190 |
| 63 | 4-$CH_3$ | H | $COCH_3$ | $CH_3$ | $CH_3$ | - | 235-237 |
| 64 | 4-$CH_3$ | 6-Cl | $CH_3$ | H | H | - | solide amorphe |
| 65 | 4-$CH_3$ | 6-Cl | $CH_3$ | H | $CH_3$ | - | solide amorphe |
| 66 | 4-$CH_3$ | 6-Cl | $CH_3$ | $CH_3$ | $CH_3$ | - | 224-225 |
| 67 | 4-$CF_3$ | H | $CH_3$ | H | H | - | 295-303 (d) |
| 68 | 4-$CF_3$ | H | $CH_3$ | H | $CH_3$ | - | 272-276 (d) |
| 69 | 4-$CF_3$ | H | $CH_3$ | $CH_3$ | $CH_3$ | - | 220-221 |
| 70 | 4-$CH_2CH_3$ | H | $CH_3$ | H | H | - | 263-265 (d) |
| 71 | 4-$CH_2CH_3$ | H | $CH_3$ | H | $CH_3$ | - | 214-215 |
| 72 | 4-$CH_2CH_3$ | H | $CH_3$ | $CH_3$ | $CH_3$ | - | 133-135 |
| 73 | 4-Cl | H | H | H | $CH_3$ | HCl | 298-303 (d) |

| N° | X | Y | $R_1$ | $R_2$ | $R_3$ | Sel | F (°C) |
|---|---|---|---|---|---|---|---|
| 74 | 4-Cl | H | H | $CH_3$ | $CH_3$ | - | 287-289 |
| 75 | 3-Cl | H | $CH_3$ | H | H | - | 266-268 (d) |
| 76 | 3-Cl | H | $CH_3$ | H | $CH_3$ | - | 268,5-271 (d) |
| 77 | 3-Cl | H | $CH_3$ | $CH_3$ | $CH_3$ | - | 222-223 |
| 78 | 4-Cl | H | $CH_3$ | H | H | - | 270-272 (d) |
| 79 | 4-Cl | H | $CH_3$ | H | $CH_3$ | - | 252-253,5 |
| 80 | 4-Cl | H | $CH_3$ | $CH_3$ | $CH_3$ | - | 229-229,5 |
| 81 | 4-Cl | H | $CH_3$ | $CH_2CH_2CH_3$ | $CH_2CH_2CH_3$ | - | 155-157 |
| 82 | 4-Cl | 6-$CH_3$ | $CH_3$ | H | H | - | >300 |
| 83 | 4-Cl | 6-$CH_3$ | $CH_3$ | H | $CH_3$ | - | 258-260 |
| 84 | 4-Cl | 6-$CH_3$ | $CH_3$ | $CH_3$ | $CH_3$ | - | 239,5-241,5 |
| 85 | 4-Cl | 6-Cl | $CH_3$ | H | H | - | 299-303 |
| 86 | 4-Cl | 6-Cl | $CH_3$ | H | $CH_3$ | - | 273-275 (d) |
| 87 | 4-Cl | 6-Cl | $CH_3$ | $CH_3$ | $CH_3$ | - | 235-235,5 |
| 88 | 4-F | H | H | H | $CH_3$ | HCl | 279-280,5 (d) |
| 89 | 4-F | H | H | $CH_3$ | $CH_3$ | HCl | 297,5-300 (d) |
| 90 | 4-F | H | $CH_3$ | H | H | - | 270,5-273,5 |

| N° | X | Y | $R_1$ | $R_2$ | $R_3$ | Sel | F (°C) |
|---|---|---|---|---|---|---|---|
| 91 | 4-F | H | $CH_3$ | H | $CH_3$ | - | 235-236,5 |
| 92 | 4-F | H | $CH_3$ | $CH_3$ | $CH_3$ | - | 224-225 |
| 93 | 4-F | 6-$CH_3$ | $CH_3$ | H | H | - | 268-270 (d) |
| 94 | 4-F | 6-$CH_3$ | $CH_3$ | H | $CH_3$ | - | 255-256 |
| 95 | 4-F | 6-$CH_3$ | $CH_3$ | $CH_3$ | $CH_3$ | - | 249-250 |
| 96 | 4-F | 6-Cl | $CH_3$ | H | H | - | 290-291 |
| 97 | 4-F | 6-Cl | $CH_3$ | H | $CH_3$ | - | 269-270 |
| 98 | 4-F | 6-Cl | $CH_3$ | $CH_3$ | $CH_3$ | - | 262-263 |
| 99 | 4-$OCH_3$ | H | $CH_3$ | H | H | - | 242-244 |
| 100 | 4-$OCH_3$ | H | $CH_3$ | H | $CH_3$ | - | 248-249,5 |
| 101 | 4-$OCH_3$ | H | $CH_3$ | $CH_3$ | $CH_3$ | - | 154-155 |
| 102 | 4-$SCH_3$ | H | $CH_3$ | H | H | - | 260-263 (d) |
| 103 | 4-$SCH_3$ | H | $CH_3$ | H | $CH_3$ | - | 264-266 (d) |
| 104 | 4-$SCH_3$ | H | $CH_3$ | $CH_3$ | $CH_3$ | - | 190,5-192 |
| 105 | 4-$SO_2CH_3$ | H | $CH_3$ | $CH_3$ | $CH_3$ | - | solide amorphe |
| 106 | 4-$CH_3$ | H | $CH_3$ | H | $C_6H_5$ | - | 268-270 (d) |
| 107 | 4-$CH_3$ | H | $CH_3$ | H | $CH_2C_6H_5$ | - | 232-238 |

| N° | X | Y | $R_1$ | $R_2$ | $R_3$ | Sel | F (°C) |
|---|---|---|---|---|---|---|---|
| 108 | 4-$CH_3$ | H | $CH_3$ | H | $CH_2CH_2N(CH_3)_2$ | - | 196-197 |
| 109 | 4-$CH_3$ | H | $CH_3$ | 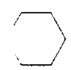 | | - | 181,5-183 |
| 110 | 4-$CO_2C_2H_5$ | H | $CH_3$ | H | $CH_3$ | - | 238-239 |
| 111 | 4-$CONH_2$ | H | $CH_3$ | H | $CH_3$ | - | 277-279 (d) |
| 112 | 4-$CO_2H$ | H | $CH_3$ | H | $CH_3$ | - | 269-270 |

Le tableau 2 qui suit illustre les structures chimiques et les propriétés physiques de quelques composés

de formule générale (IV), intermédiaires dans le procédé du schéma 1. Tous les composés sont à l'état de bases.
Dans la colonne "F (°C)", "(d)" désigne une température de fusion avec décomposition, et "(l/d)" désigne une température de fusion lente avec décomposition.

Tableau 2

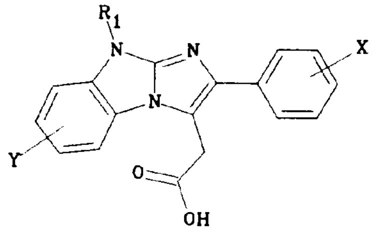

(IV)

| N° | X | Y | $R_1$ | F (°C) | |
|---|---|---|---|---|---|
| 1' | H | H | $CH_3$ | 241-244 | (d) |
| 2' | H | 6-$CH_3$ | $CH_3$ | 210-215(*) | (l/d) |
| 3' | H | 6-Br | $CH_3$ | 245-250 | |
| 4' | H | 6-Cl | $CH_3$ | 245-250 | (l/d) |
| 5' | H | 7-Cl | $CH_3$ | 230-232 | (l/d) |
| 6' | H | 6-F | $CH_3$ | 252-254 | (l/d) |
| 7' | H | 6-$OCF_3$ | $CH_3$ | solide amorphe | |
| 8' | H | 6,7-$(CH_3)_2$ | $CH_3$ | 220 | (l/d) |
| 9' | 2-$CH_3$ | H | $CH_3$ | 262-264 | (d) |
| 10' | 3-$CH_3$ | H | $CH_3$ | 212-218 | (d) |
| 11' | 4-$CH_3$ | H | $CH_3$ | 250-254 | (d) |
| 12' | 4-$CH_3$ | H | $CH_2CH_3$ | 216-218 | (d) |
| 13' | 4-$CH_3$ | H | $CH_2C_6H_5$ | 233-239 | (l/d) |
| 14' | 4-$CH_3$ | 6-Cl | $CH_3$ | solide amorphe | |
| 15' | 4-$CF_3$ | H | $CH_3$ | 250-253 | (d) |
| 16' | 4-$CH_2CH_3$ | H | $CH_3$ | 250-252 | (d) |

| N° | X | Y | $R_1$ | F (°C) | |
|---|---|---|---|---|---|
| 17' | 3-Cl | H | $CH_3$ | 210-215 | (d) |
| 18' | 4-Cl | H | $CH_3$ | 252-255 | (d) |
| 19' | 4-Cl | 6-$CH_3$ | $CH_3$ | 265-270 | (d) |
| 20' | 4-Cl | 6-Cl | $CH_3$ | 260-265 | (d) |
| 21' | 4-F | H | $CH_3$ | 261-266 | (l/d) |
| 22' | 4-F | 6-$CH_3$ | $CH_3$ | 223-225 | (d) |
| 23' | 4-F | 6-Cl | $CH_3$ | 258-260 | (d) |
| 24' | 4-$OCH_3$ | H | $CH_3$ | 220-222 | (d) |
| 25' | 4-$SCH_3$ | H | $CH_3$ | 249-252 | (d) |

(*) Le composé numéro 2' présente un premier point de fusion à 150-155°C, au delà duquel il recristallise.

Le tableau 3 qui suit illustre les structures chimiques et les propriétés physiques de quelques composés de formule générale (VI), intermédiaires dans le procédé du schéma 2. Tous les composés sont à l'état de bases.
Dans la colonne "F (°C)", "(d)" désigne une température de fusion avec décomposition.

Tableau 3

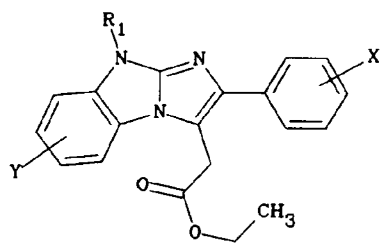

(VI)

| N° | X | Y | $R_1$ | F (°C) | |
|---|---|---|---|---|---|
| 1" | H | H | H | 220-222 | |
| 2" | H | 6-F | H | solide amorphe | |
| 3" | 4-$CH_3$ | H | H | 234-237 | (d) |
| 4" | 4-Cl | H | H | 260-261 | |
| 5" | 4-F | H | H | 245-247 | (d) |

Les composés de l'invention ont été soumis à des essais pharmacologiques qui ont mis en évidence leur

intérêt comme substances à activités thérapeutiques.

Etude des liaisons membranaires vis-à-vis des récepteurs $\omega_1$ (benzodiazépiniques de type 1) et $\omega_2$ (benzodiazépiniques de type 2).

L'affinité des composés pour les récepteurs $\omega_1$ du cervelet et $\omega_2$ de la moëlle épinière a été déterminée selon une variante de la méthode décrite par S. Z. Langer et S. Arbilla dans *Fund. Clin. Pharmacol.*, **2**, 159-170 (1988), avec utilisation de $^3$H-flumazenil au lieu de $^3$H-diazepam comme radioligand. On homogénéise le tissu du cervelet ou de la moëlle épinière pendant 60 s dans 120 ou 30 volumes, respectivement, de tampon glacé (50 mM Tris/HCl, pH 7,4, 120 mM NaCl, 5 mM KCl) puis, après dilution à 1/3, on fait incuber la suspension avec du $^3$H-flumazenil (activité spécifique 78 Ci/mmole, New England Nuclear) à une concentration de 1 nM et avec les composés de l'invention à différentes concentrations, dans un volume final de 525 µl. Après 30 minutes d'incubation à 0°C on filtre les échantillons sous vide sur des filtres Whatman GF/B® et on les lave immédiatement avec du tampon glacé. La liaison spécifique du $^3$H-flumazenil est déterminée en présence de diazépam 1 µM non marqué. On analyse les données selon les méthodes usuelles et on calcule la concentration $CI_{50}$, concentration qui inhibe de 50% la liaison du $^3$H-flumazenil.
Les $CI_{50}$ des composés de l'invention se situent, dans ces essais, entre 1 et 300 nM.

Etude de l'activité hypnotique.

L'activité sédative ou hypnotique des composés a été déterminée par l'observation de leur action sur l'électrocorticogramme du rat, selon la méthode décrite par H. Depoortere, *Rev. E.E.G. Neurophysiol.*, **10**, 3, 207-214 (1980) et par H. Depoortere et M. Decobert, *J. Pharmacol. (Paris)*, **14**, 2, 195-265 (1983).
Les produits à étudier ont été administrés par voie intrapéritonéale à doses croissantes. Ils induisent des tracés de sommeil à des doses allant de 0,1 à 30 mg/kg.

Etude de l'activité anxiolytique.

L'activité anxiolytique est évaluée chez le rat dans le test de conflit de prise de boisson, selon la méthode décrite par J. R. Vogel, B. Beer et D. E. Clody dans *Psychopharmacologia* (Berl.), **21**, 1-7 (1971).
Après une diète hydrique de 48h, le rat est placé dans une enceinte isolée du bruit et équipée d'une pipette d'eau reliée à un anxiomètre délivrant un léger choc électrique tous les 20 coups de langue. Le nombre de chocs reçus est automatiquement compté pendant 3 minutes, et permet d'évaluer l'activité anxiolytique des composés testés. Les résultats sont exprimés par la dose efficace minimale (DEM), dose qui produit une augmentation significative du nombre de chocs reçus, par rapport au nombre observé chez les animaux témoins.
Les DEM des composés de l'invention se situent, dans cet essai, entre 1 et 50 mg/kg par la voie intrapéritonéale ou orale.

Etude de l'activité anticonvulsivante.

Activité vis-à-vis des convulsions maximales induites chez la souris par électrochoc ou par injection de pentétrazol.

Le protocole de cet essai est décrit par E. A. Swinyard et J. H. Woodhead dans *Antiepileptic Drugs,* Raven Press, New York, 111-126 (1982).
30 minutes après administration intrapéritonéale du composé à tester, on note le nombre de souris présentant des convulsions (extensions des pattes arrière), soit immédiatement après application d'un courant électrique (0,4 s, 60 mA, 50 Hz) à l'aide d'électrodes transcornéennes, soit pendant les 30 minutes qui suivent l'injection sous-cutanée de pentétrazol (125 mg/kg). Les résultats sont exprimés par la $DA_{50}$, dose qui protège 50% des animaux, calculée selon la méthode de J. T. Lichtfield et F. Wilcoxon *(J. Pharm. Exp. Ther.*, **96**, 99-113 (1949)) à partir de 3 ou 4 doses administrées chacune à un groupe de 8 à 10 souris.
Les $DA_{50}$ des composés de l'invention se situent, dans cet essai, entre 1 et 100 mg/kg par la voie intrapéritonéale.

Etude de l'activité anticonvulsivante.

Activité vis-à-vis des convulsions induites chez la souris par l'isoniazide.

L'activité intrinsèque des composés est déterminée par le temps de latence d'apparition des convulsions induites par l'administration sous-cutanée d'isoniazide (800 mg/kg) simultanément avec le composé à tester, injecté par voie intrapéritonéale, selon le protocole décrit par G. Perrault, E. Morel, D. Sanger et B. Zivkovic dans *Eur. J. Pharmacol.*, **156**, 189-196 (1988). Les résultats sont exprimés par la $DA_{50}$, dose qui produit 50% de l'effet maximal, par rapport aux animaux témoins, déterminée à partir de 3 ou 4 doses administrées chacune à un groupe de 8 à 10 souris.
Les $DA_{50}$ des composés de l'invention se situent, dans cet essai, entre 1 et 50 mg/kg par la voie intrapéritonéale et, selon les composés, l'effet maximal peut aller jusqu'à 350%.

Les résultats des essais effectués sur les composés de l'invention montrent que, *in vitro,* ils déplacent le $^3$H-flumazénil de ses sites de liaison spécifiques au niveau du cervelet et de la moëlle épinière ; par conséquent ils présentent une affinité pour les sites $\omega_1$ et $\omega_2$ (benzodiazépiniques de type 1 et de type 2) situés au sein du complexe macromoléculaire $GABA_A$-sites $\omega$-canal chlorure.
*In vivo* ils se comportent comme des agonistes complets ou partiels, ou comme des antagonistes vis-à-vis de ces récepteurs.
Ils possèdent des propriétés hypnotiques, anxiolytiques et anticonvulsivantes et, par conséquent, peuvent être utilisés pour le traitement d'affections liées aux désordres de la transmission GABAergique, tels que l'anxiété, les troubles du sommeil, l'épilepsie, la spasticité, les contractures musculaires, les troubles cognitifs, les troubles du sevrage vis-à-vis de l'alcoolisme, etc. Enfin, ils peuvent être utilisés dans la prémédication et comme anesthésiques généraux pour l'induction et/ou le maintien de l'anesthésie, ou comme anesthésiques locaux, éventuellement associés à d'autres anesthésiques et/ou des myorelaxants et/ou des analgésiques.

A cet effet ils peuvent être présentés sous toutes formes galéniques, associés à des excipients appropriés, pour l'administration entérale ou parentérale, par exemple sous forme de comprimés, dragées, gélules, capsules, solutions ou suspensions buvables ou injectables, suppositoires, etc, dosés pour permettre une administration journalière de 1 à 1000 mg de substance active.

## Revendications

**1.** Composé répondant à la formule générale (I)

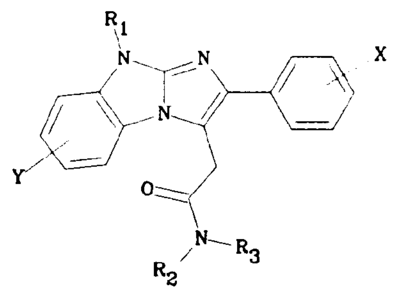

(I)

dans laquelle
X représente un ou plusieurs atomes ou groupes choisis parmi l'hydrogène, le fluor, le chlore, le brome et les groupes ($C_1$-$C_3$)alkyle, trifluorométhyle, ($C_1$-$C_3$) alcoxy , ($C_1$-$C_3$)alkylthio, méthylsulfonyle, cyano, éthoxycarbonyle, aminocarbonyle et carboxy,
Y représente un ou plusieurs atomes ou groupes choisis parmi l'hydrogène, le fluor, le chlore, le brome et les groupes ($C_1$-$C_4$) alkyle, trifluorométhyle, méthoxy et trifluorométhoxy, $R_1$ représente un atome d'hydrogène, un groupe ($C_1$-$C_3$)alkyle, un groupe phénylméthyle, un groupe 2-phényléthyle, un groupe acétyle ou un groupe ($C_1$-$C_3$)alcoxycarbonyle,
$R_2$ et $R_3$ représentent chacun, indépendamment l'un de l'autre, soit un atome d'hydrogène, soit un groupe ($C_1$-$C_5$)alkyle linéaire ou éventuellement ramifié ou cyclique, et éventuellement substitué par un ou plusieurs atomes de fluor, par un groupe méthoxy, par un groupe phénoxy, par un groupe diméthylamino, par un groupe phényle ou par un groupe imidazol-4-yle, soit un groupe prop-2-ényle, soit un groupe prop-2-ynyle, soit un groupe phényle, soit un groupe 1-(phénylméthyl)pipéridin-4-yle, soit un groupe 1-[(cyclohexen-1-yl)méthyl]pipéridin-4-yle,
ou bien
$R_2$ et $R_3$ forment ensemble, et avec l'atome d'azote qui les porte, un groupe pyrrolidin-1-yle, 3-éthoxypyrrolidin-1-yle, pipéridin-1-yle, 4-(phénylméthyl)pipéridin-1-yle, spiro(dioxolane-2,4'-pipéridin)-1'-yle, 3-(phénoxyméthyl)pipéridin-1-yle, 4-(phénoxyméthyl)pipéridin-1-yle, hexahydroazépin-1-yle, 4-méthylpipérazin-1-yle, 4-(phénylméthyl)pipérazin-1-yle, morpholin-4-yle ou thiomorpholin-4-yle,
à l'état de base libre ou de sel d'addition à un acide.

**2.** Composé selon la revendication 1, caractérisé en ce que X est en position 4 et représente un atome d'hydrogène ou de fluor ou un groupe méthyle.

**3.** Composé selon la revendication 1, caractérisé en ce que Y est en position 6 et représente un atome d'hydrogène ou de fluor ou un groupe méthyle.

**4.** Composé selon la revendication 1, caractérisé en ce que $R_1$ représente un atome d'hydrogène ou un groupe méthyle.

**5.** Composé selon la revendication 1, caractérisé en ce que $R_2$ et $R_3$ représentent chacun, indépendamment l'un de l'autre, un atome d'hydrogène ou un groupe méthyle.

**6.** Procédé de préparation de composés selon la revendication 1, caractérisé en ce que
. ou bien
on fait réagir un dérivé de 9*H*-imidazo[1,2-*a*]benzimidazole de formule générale (II)

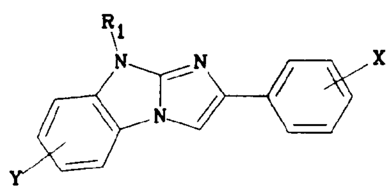

(II)

(dans laquelle X, Y et $R_1$ sont tels que définis dans la revendication 1) avec le *N,N*-diméthylglyoxamide, dans un solvant protique, à une température de 20 à 80°C,
puis on traite le dérivé d'α-hydroxyacétamide ainsi obtenu, de formule générale (III)

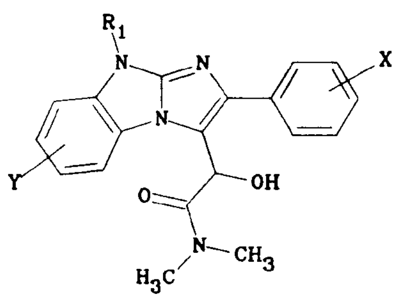

(III)

avec un polyhalogénure d'acide sulfurique ou phosphorique, dans un solvant inerte, à une température de 20 à 80°C, pour former le dérivé de α-halogénoacétamide correspondant, puis on fait réagir ce dernier soit avec un agent réducteur tel qu'un hydrure alcalin simple ou complexe, dans un solvant protique, ou dans un solvant inerte miscible à l'eau, à une température de -40 à 40°C, soit avec un agent réducteur tel qu'un hyposulfite ou un dithionite alcalin, ou encore avec l'hydroxyméthylsulfoxylate de sodium, dans un solvant inerte, éventuellement en présence d'un cosolvant inerte miscible à l'eau, à une température de 20 à 40°C, pour obtenir un dérivé de *N,N*-diméthylacétamide de formule générale (Ia)

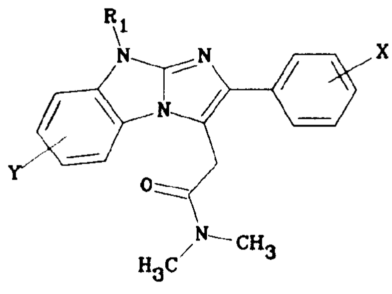

(Ia)

qui correspond à la formule générale (I) lorsque $R_2$ et $R_3$ représentent chacun un groupe méthyle, puis, si on désire préparer un composé de formule générale (I) dans laquelle $R_2$ et $R_3$ ne représentent pas chacun un groupe méthyle, on transforme le composé de formule générale (Ia) en acide de formule générale (IV)

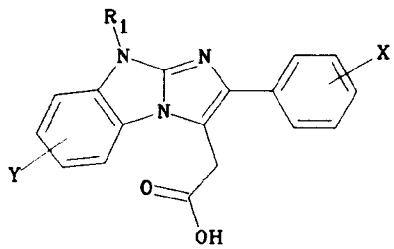

(IV)

par hydrolyse au moyen d'une base forte, dans un solvant protique, en présence d'eau,
puis on fait réagir l'acide de formule générale (IV) avec le *N,N'*-carbonyldiimidazole, dans un solvant inerte, à une température de 20 à 50°C, pour obtenir l'imidazolide correspondant, et finalement on traite ce dernier avec une amine de formule générale $HNR_2R_3$ (dans laquelle $R_2$ et $R_3$ sont tels que définis dans la revendication 1) à une température de 0 à 25°C,
. ou bien
on fait réagir un dérivé de 9*H*-imidazo[1,2-*a*]benzimidazole de formule générale (II)

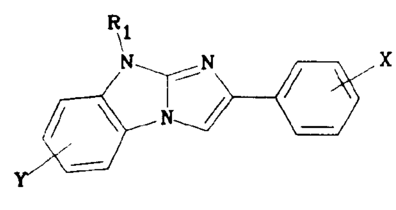

(II)

(dans laquelle X, Y et $R_1$ sont tels que définis dans la revendication 1) avec le glyoxylate d'éthyle (que l'on prépare *in situ* au moyen de 2,2-diéthoxyacétate d'éthyle) dans un solvant protique, à une température de 20 à 80°C, puis on traite le dérivé d'α-hydroxyacétate d'éthyle de formule générale (V) ainsi obtenu

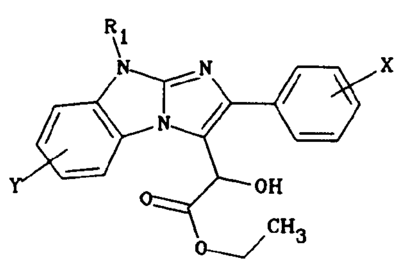

(V)

avec un polyhalogénure d'acide sulfurique ou phosphorique, dans un solvant inerte, à une température de 20 à 80°C, pour former le dérivé de α-halogénoacétate d'éthyle correspondant, puis on fait réagir ce

dernier soit avec un agent réducteur tel qu'un hydrure alcalin simple ou complexe, dans un solvant protique, ou dans un solvant inerte miscible à l'eau, à une température de -40 à 40°C, soit avec un agent réducteur tel qu'un hyposulfite ou un dithionite alcalin, ou encore avec l'hydroxyméthylsulfoxylate de sodium (Rongalite®), dans un solvant inerte, éventuellement en présence d'un cosolvant inerte miscible à l'eau, à une température de 20 à 40°C, pour obtenir un composé de formule générale (VI)

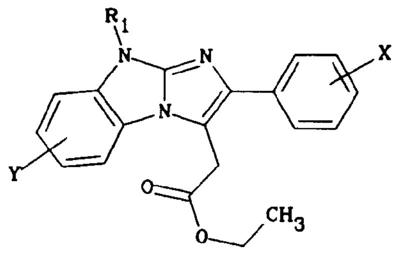

(VI)

que l'on fait ensuite réagir avec un excès d'amine de formule générale $HNR_2R_3$ (dans laquelle $R_2$ et $R_3$ sont tels que définis dans la revendication 1) dans un solvant protique, à une température de 0 à 70°C.

**7.** Médicament caractérisé en ce qu'il consiste en un composé selon l'une des revendications 1 à 5.

**8.** Composition pharmaceutique caractérisée en ce qu'elle contient un composé selon l'une des revendications 1 à 5, associé à un excipient.

**9.** Composé de formule générale (III), tel que défini dans la revendication 6, à titre d'intermédiaire nécessaire dans le procédé selon la revendication 6.

**10.** Composé de formule générale (IV), tel que défini dans la revendication 6, à titre d'intermédiaire nécessaire dans le procédé selon la revendication 6.

**11.** Composé de formule générale (VI), tel que défini dans la revendication 6, à titre d'intermédiaire nécessaire dans le procédé selon la revendication 6.

Office européen des brevets

# RAPPORT DE RECHERCHE EUROPEENNE

Numero de la demande
EP 94 40 0057

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.5) |
|---|---|---|---|
| A | EP-A-0 172 097 (SYNTHELABO) 19 Février 1986<br>* page 14, ligne 21 - ligne 32; revendication 1 *<br>----- | 1,7 | C07D487/04<br>A61K31/415<br>A61K31/445<br>A61K31/495<br>A61K31/535<br>A61K31/54<br>A61K31/55<br>C07D519/00<br>//(C07D487/04, 235:00, 235:00), (C07D519/00, 498:00,487:00) |
| | | | **DOMAINES TECHNIQUES RECHERCHES (Int.Cl.5)** |
| | | | C07D<br>A61K |

Le présent rapport a été établi pour toutes les revendications

| Lieu de la recherche | Date d'achèvement de la recherche | Examinateur |
|---|---|---|
| LA HAYE | 31 Mars 1994 | Alfaro Faus, I |

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)